# EUROPÄISCHE PATENTANMELDUNG

(11) **EP 2 100 603 A1**
(43) Veröffentlichungstag der Anmeldung: **16.09.2009**
(21) Anmeldenummer: 06849634.8
(22) Anmeldetag: 29.11.2006
(51) Int. Cl.: A61K 31/194, A61K 31/14, A61P 35/00

(54) **VERWENDUNG VON MALACHITGRÜN IN FORM EINES ARZNEIMITTELS ZUR BEHANDLUNG VON MALIGNEN NEOPLASIEN**

(71) Anmelder: Germanov, Evgeny Pavlovich, Moscow 129085 (RU); Kutushov, Mikhail Vladimirovich, Moscow, 125414 (RU)
(72) Erfinder: KUTUSHOV, Mikhail Vladimirovich, Moscow 125414 (RU)
(74) Vertreter: Jeck, Anton
(86) Internationale Anmeldenummer: PCT/RU2006/000639
(87) Internationale Veröffentlichungsnummer: WO 2008/066402

(57) **Zusammenfassung**

Die Erfindung betrifft die Anwendung von Malachitgrün als Arzneimittel zur Behandlung von malignen Neubildungen, das in Form von ANHYDROOXALAT TETRAMETHYLDIAMINTRIPHENYLKARBINOL (ANHYDROOXALATE TETRAMETHYLDIAMISHTRIPHENOLCARBINOL) [(C₂₃H₂₅N₂)-(C₂HO₄)₂-C₂H₂O₄ mit folgender Bauformel: 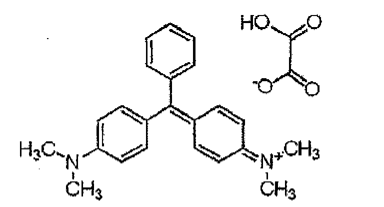 als Mittel zur Behandlung von malignen Neubildungen mit einer Dosierung des Arzneimittels von 1 mg bis 2 g pro Einnahme eingesetzt wird. Damit ist ein Heilmittel geschaffen, das ein breites Spektrum der Heilwirkung bei unterschiedlichen Geschwulsterkrankungen besitzt.

## Beschreibung

Die Erfindung betrifft die Anwendung von Malachitgrün als Arzneimittel zur Behandlung von malignen Neubildungen.

Die vorgeschlagene Erfindung kann in der Medizin, nämlich als Breitspektrumarzneimittel, das zur Behandlung von Geschwulstkrankheiten eingesetzt und als OF3 bezeichnet wird, angewendet werden.

Es ist das Heilmittelpräparat Fluororacil bekannt, welches ein weißes oder gelbliches Kristallpulver darstellt, das im Wasser und im Alkohol schwachlöslich ist. Dieses Präparat ist ein Antimetabolit. Seine Antitumoraktivität wird dadurch festgelegt, dass es sich in den Krebszellen in einen kompetitiven Hemmstoff gegenüber dem Ferment verwandelt, welches in der Nukleinsäuresynthese mitwirkt (s. z. B. M.D. Mashkovsky, Heilmittel, Moskau, OOO Novaja Volna, Herausgeber S.B. Divov, 2002, Band 2, S. 425).

Dieses Präparat wird in Form von intravenösen Injektionen bei inoperablem und wiederholtem Magenkrebs, Dickdarmkrebs und Mastdarmkrebs, Mamma- und Eierstockkarzinom, sowie Pankreaskrebs angewendet. Jedoch weist dieses Präparat eine hohe Toxizität auf. Bei seiner Anwendung können auch Blutbildungsinhibition, Diarrhoe, Appetitminderung, Erbrechen, geschwürige Stomatitis entstehen Außerdem ist dieses Präparat bei allgemeinen schweren Zuständen der Kranken, bei Magen- und Zwölffingerdarmgeschwüren sowie bei ausgeprägten funktionellen Leberversagen nicht anwendbar und daher untersagt.

Der nächste Stand der Technik und der Prototyp ist das Präparat Rheaferon, welches das rekombinante α2-Interferon darstellt. Das rekombinante α2-Interferon wird durch den bakteriellen Stamm von Pseudomonas erzeugt, im dessen genetischen Apparat das Gen von menschlichem leukozytärem α2-Interferon integriert wird. Dieses Präparat wird in Form von einem porösen Pulver hergestellt. Die Wasserlösung wird zur intramuskulären oder subkutanen Injektion angewendet (s., z. B. M.D. Mashkovsky "Heilmittel", Moskau, OOO Novaja Volna, Herausgeber S.B. Divov, 2002, Band 2, S. 323 - 324).

Das Präparat weist virentötende, immunomodulierende und antiblastomatöse Aktivität auf. Es ist bei der Behandlung von Virushepatitis effektiv. Es wird auch bei der Behandlung von Haarzellenleukämie, Kaposis Sarkom (beim AIDS-Hintergrund), bei Nierenkrebs, metastatischem Melanom usw. angewendet. Jedoch sind bei der Anwendung von Rheaferon Fieberschauer, allgemeines Unwohlsein, allergische Hautreaktionen, Leuko- und Thrombozytopenie möglich. Die Anwendung von diesem Heilmittel ist auch bei allergischen Erkrankungen, ausgeprägten Leber- und Nierenkrankheiten sowie bei Schwangerschaft untersagt.

Es ist Aufgabe der Erfindung ein Heilmittel zu schaffen, welches ein breites Spektrum der Heilwirkung bei unterschiedlichen Geschwulstkrankheiten aufweist.

Das technische Ergebnis, welches die Lösung der gestellten Aufgabe sicherstellt, besteht in der Anwendung von einer breit zugänglichen und kostengünstigen Substanz als Mittel zur Behandlung von Geschwulstkrankheiten. Diese Substanz muß nicht toxisch sein und darf keine Nebenwirkungen bei ihrer Anwendung in pharmazeutisch zulässigen Dosen, bei Kürzung der Behandlungsdauer und bei der Erhöhung der Behandlungseffektivität haben.

Die Erfindung besteht in der Anwendung von Malachitgrün in Form von ANHYDROOXALAT TETRAMETHYLDIAMINTRIPHENYLKARBINOL (ANHYDROOXALATE TETRAMETHYLDIAMISHTRIPHENOLCARBINOL) [(C₂₃H₂₅N₂)-(C₂HO₄)]₂-C₂H₂O₄ mit folgender Bauformel: 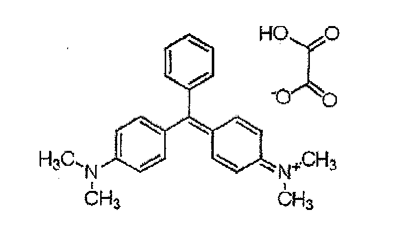 als Mittel zur Behandlung von malignen Neubildungen mit einer Dosierung von Arzneimittel von 1 mg bis 2 g pro Einnahme.

Das Malachitgrün kann als Wasserlösung oder in einer physiologischen Kochsalzlösung oder aufgelöst in Alkohol angewendet werden. In verschiedenen Anwendungsfällen wird die Lösung von Malachitgrün peroral vor oder nach dem Essen eingenommen, oder die Lösung von Malachitgrün wird rektal eingeführt, oder die 1%ige Lösung von Malachitgrün wird intravenös injektiert, oder das Malachitgrün wird rektal in Form von Zäpfchen angewendet, oder das Malachitgrün wird als Komponente einer 1 - 5 % Salbe angewendet.

Der pflanzliche Farbstoff Malachitgrün (Malachite Green oxalate salt) oder ANHYDROOXALAT TETRAMETHYLDIAMINTRIPHENYLKARBINOL hat die entsprechende chemische Bezeichnung: (dimethylamino)benzhydrylidene] cyclohexa-2,5-dien-1-ylidene] dimethylammonium] oxalate, dioxalate; N,N,N',N'-Tetramethyl-4,4'- diaminotriphenylcarbenium oxalate; C.I. 42000; Malachite green oxalate; Victoria Green B). Hersteller: REAGENA, Finnland. Synonyme: Benzoylgrün, Bittermandelölgrün, Berggrün, Malachitgrün. Das ist ein synthetischer Diaminotriphenilmethanfarbstoff. Die grünen oder gelben Kristalle mit Metallglanz sind in Wasser, in Alkohol und in physiologischer Kochsalzlösung gut lösbar.

Momentan wird das Maladhitgrün in der Mikroskopie bei Vitalfärbung von Zellkernen, zur Färbung von Erythrozyten, Spulwurmeiern sowie als Heilmittel bei der Behandlung von Aquarienfischen gegen Infektionskrankheiten angewendet.

Die Voraussetzungen für die Einwirkung von Farbstoffen auf die Strukturen von bösartigen Zellen sind wie folgt. In onkologischen Zellen (Krebszellen) sind die Polarisation und Anisotropie und folglich das spontane Zellenleuchten gestört. Die Fotoaktivität der Strukturen in der Zellenlebenfunktion hat eine der Hauptrollen. Viele biochemische Reaktionen und Zellenmitose werden von den photochemischen Reaktionen begleitet, was als quantenmechanische Erscheinungen beschrieben werden kann. Sowohl Krebs als auch viele andere Krankheiten können behandelt werden, indem auf diese Mechanismen eingewirkt wird. S. das Buch "Krebs ist heilbar", M.V. Kutushov. Herausgeber V. Sekachev, Moskau. 2005. S. 393.

Beim Krebs treten räumliche Störungen des Zellengefüges, und vor allem eine unrichtige Proteinenanordnung auf. Aus der Physik ist bekannt, dass die Größe der Grenzpolarisation mit der Molekülsymmetrie zusammenhängt. Bei krebsbedingter Entartung der Zellengefüge geht die Starrheit der Verbindung zwischen Oszillatoren und Molekül verloren. Deswegen nimmt der Anisotropiegrad des Moleküls und folglich der Polarisationsgrad ab. Dieser Umstand bedingt, dass sogar bei ziemlich anisotropen Molekülen der Polarisationsgrad den theoretischen Grenzwert nicht erreicht. Folglich, wenn der zulässige Farbstoff in ein solches System eingeführt wird, so muss er den Polarisationsgrad ausgleichen, die gestörte Dissymmetrie und Anisotropie wiederherstellen. Die organischen Farbstoffe weisen mehr oder weniger ein Strahlungsgenerationsvermögen auf. In den Generationsspektren der meisten Farbstoffe wird eine Reihe von scharfen Äquidistantlinien beobachtet. Der Abstand zwischen diesen Linien hängt von den Eigenschaften des Resonators ab. Der Resonator hat viele Reflexionsflächen. Wenn als Resonator zum Beispiel die Zelle selbst und ihr Gefüge angenommen werden, so werden die Resonanzeigenschaften je nach Norm und Pathologie variieren. Beim Krebs sind die Zellen- und Gewebegefüge sehr grob gestört.

Alle Farbstoffmoleküle weisen ein flaches Gerippe auf. Die einzelnen Gruppen von Radikalen (Reste) ragen nur selten aus der gemeinsamen Ebene hinaus. Das Malachitgrün als ein Triphenylmethanfarbstoff genauso wie einige andere aufgelöste Farbstoffe fluoreszieren nicht, weil ihre Struktur eine Rotation der Radikalgruppen um die Konjugationsverbindungsketten zulässt. Bei Festlösungen (Flüssigkristallen) weisen sie ein intensives Leuchten auf. Die Krebsproteine befinden sich in festen würfelförmigen Syngonien. Deswegen leuchten solche Farbstoffe darin und fungieren als Quantengeneratoren. Gleichzeitig haben die Farbstoffe eine hohe Aufnahmefähigkeit im sichtbaren Spektralgebiet. Deswegen bringen die Farbstoffe die ursprünglichen engen Absorptionsspektren oder mit anderen Worten die äquidistanten Eigenschaften solcher Strukturen zurück, indem diese Farbstoffen mit solchen Strukturen zusammenwirken.

Somit kann man schlussfolgern, dass die Farbstoffe selbst oder sie in Verbindung mit den Zellengefügen fähig sind, jene Wellenlänge zu dämpfen, welche durch die Krebsstrukturen für ihre Lebensaktivität benutzt werden. Dadurch wird ihr Verfall verursacht. Diese Eigenschaften der Farbstoffe sind in der Krebstherapie festgelegt. Die symmetrischen und asymmetrischen Farbstoffe haben eine überzweizählige Symmetrie. Es ist bekannt, dass die Grenzpolarisation der symmetrischen und asymmetrischen Farbstoffe bei der Änderung der Wellenlänge des angeregten Lichts innerhalb des Langwellenabsorptionsbandes praktisch unverändert bleibt. Bei Triphenylmethanfarbstoffen ändert sich der Polarisationsgrad akut bei der Änderung der Wellenlänge des angeregten Lichts innerhalb dieses Langwellenabsorptionsbandes. Deswegen wurde erfahrungsmäßig ein ungiftiger Farbstoff Malachitgrün gewählt. Malachitgrün ist zur Generation und Selbstgeneration der Photonen mit der erforderlichen Wellenlänge fähig. Das Malachitgrün wechselt von gelb auf blau innerhalb eines sehr engen Bereichs sogar bei einer sehr geringen Photonenmenge (Beleuchtung). Diese Eigenschaft, welche keinem anderen Farbstoff in solchem Umfang eigen ist, entspricht allen Anforderungen, die an die von uns in der Krebsbehandlung eingesetzten Farbstoffe gestellt werden. Dieser Mechanismus liegt der selektiven Unterdrückung der Zellenstrukturen zugrunde.

Die durchgeführten Forschungen haben gezeigt, dass das Malachitgrün als Heilmittel in Form von Oxalat antiblastomatöse Wirkung hat. Diese Wirkung zeigt sich auch in Bezug auf Leukosen mittels einer objektiven Manifestation des therapeutischen antiblastomatösen Effekts. Dies wurde durch Instrumentallaboruntersuchungen nachgewiesen. Es wurde eine Blutuntersuchung der Patienten sowie die Messungen der Tumorgröße vor und nach der Einnahme des jeweiligen Präparats Malachitgrün durchgeführt.

Das Präparat kann oral, rektal bzw. intravenös eingenommen werden. Dafür soll es aufgelöst, gefiltert, umkristallisiert und sterilisiert werden.

Das Heilmittelpräparat auf Malachitgrün-Basis kann oral in Form von Pulver oder Tabletten beim oder nach dem Essen eingenommen werden. Es kann auch per rectum in Form von Zäpfchen, extern in Form von Suspensionen und Salben benutzt werden. Dabei verursacht seine Anwendung innerhalb von einem breiten Bereich von Dosierungen (1 mg bis 2 g) keine allergischen Reaktionen und sonstige Nebenwirkungen. Seine Effektivität (bei der Behandlung von entsprechenden Erkrankungen) ist mindestens so hoch wie die des als Prototyp gewählten Präparats.

Die Ergebnisse der Untersuchungen des Wirkungsmechanismus des vorgeschlagenen Heilmittels sind in Beispielen 1 bis 5 dargelegt. Die Anwendbarkeit des Heilmittels zur Behandlung von besagten Krankheiten wird durch Beispiele 6 - 15 nachgewiesen.

### Beispiel 1

Das Heilmittel, verdünnt bis 10⁻⁵ mMol/l, in Form von Malachitgrünlösung wurde in die Prüfgläser der Versuchsgruppe mit Krebszellen PC-3 (Prostatakarzinom) in einer Pufferlösung (10 ml) eingeführt.

Außerdem wurden in die Prüfgläser mit dem gleichen Krebszellenpool - in derselben Verdünnung - jeweils solche Präparate wie Adriamycin, Rheaferon sowie eine physiologische Kochsalzlösung eingeführt.

Dabei wurde auch eine Kontrollgruppe aus der gleichen Anzahl von Prüfgläser und mit denselben Komponenten bis auf die Krebszellen gebildet. Anstatt dieser Zellen wurden Fibroblase in die Prüfgläser dieser Gruppe gesetzt.

Nach der Temperierung innerhalb von 72 Stunden bei einer Temperatur von 37 °C wurde eine Inhaltsanalyse der oben genannten Prüfgläser vorgenommen.

Nach dem Studium der erfassten Ergebnisse wurde Folgendes festgestellt:

Die Mitochondrialmembrane aus den Prüfgläsern der Versuchsgruppe mit dem Heilmittel wurden praktisch völlig zerstört. Dabei war das Bild bei allen Varianten der Anwendung des Heilmittels gleich. In den Prüfgläsern dieser Gruppe mit Adriamycin wurde eine Schwellung von Membranen festgestellt. In den Prüfgläsern dieser Gruppe mit Präparat Rheaferon wurde die Schwellung von Membranen mit einer teilweisen (ca. 60 %) Zerstörung festgestellt. Bei den Zellen aus dem Prüfglas mit der physiologischen Kochsalzlösung wurden keine Änderungen erkannt.

Die Mitochondrialmembrane aus den Prüfgläsern der Kontrollgruppe waren angeschwollen, jedoch war ihre Integrität nicht betroffen.

Die erfassten Angaben zeugen davon, dass das vorgeschlagene Heilmittel zur Zerstörung der Mitochondrialmembrane der Krebszellen geführt hat. Allerdings ändert dies nicht die Struktur der Normalzellen. Mit anderen Worten, das Heilmittel erhöht die Fähigkeit der Zellenmitochondrien, die Fermente zu erzeugen, welche den die Apoptosis der Krebszellen herbeiführt.

### Beispiel 2

Das Heilmittel, verdünnt bis 10⁻⁵ mMol/l, in Form von Malachitgrünlösung wurde in 4 Prüfgläser der Versuchsgruppe mit Krebszellen MCF-7 (Mammaadenokarzinom) in einer Pufferlösung (10 ml) eingeführt.

Kontrollgruppe: Jedes Prüfglas enthielt das gleiche Krebszellenpool. Das eine Prüfglas wurde mit physiologischer Kochsalzlösung, und die anderen Prüfgläser mit den anderen Präparaten Adriamycin und Rheaferon ergänzt.

In den Prüfgläsern beider Gruppen wurden Titer von Zytochrom-C festgestellt, die 1 : 14000 betrugen.

Nach der Temperierung innerhalb von 24 Stunden bei einer Temperatur von 37 °C wurde Inhaltsanalyse der oben genannten Prüfgläser vorgenommen.

Nach dem Studium der erfassten Ergebnisse wurde Folgendes festgestellt:

Der Titer von Zytochrom-C aus den Prüfgläsern der Versuchsgruppe mit eingeführtem Heilmittel betrug im Prüfglas mit Malachitgrün 1 : 2000.

In den Prüfgläsern mit Adriamycin und Rheaferon betrug der Titer jeweils 1 : 12000 und 1 : 9600. Im Prüfglas mit der physiologischen Kochsalzlösung wurden keine besonderen Titeränderungen festgestellt.

Diese Ergebnisse ermöglichen die Annahme, dass das vorgeschlagene Heilmittel zur Freisetzung vom "aggressiven" Protein von Zytochrom-C aus den Mitochondrialmembranen der Krebszellen beiträgt. Dieses Protein löst den Mechanismus der DNS aus, was die Apoptosis der Krebszellen bedingt.

### Beispiel 3

In das Prüfglas (10 ml) mit Transthyretin (Protein) des Blutplasmas (pH-7,0), Konzentration 0,05 mMol/l, wurden 4 Tropfen einer 0,1%igen Wasserlösung von Malachitgrün gegeben.

Das Prüfglas wurde in einen Wärmeschrank (Inkubator) mit einer Temperatur von 37 °C gesetzt. Nach 15 Minuten wurden die Konzentration von Transthyretin a und das pH des Plasmas gemessen.

Das Transthyretin ließ sich nicht erkennen, pH beträgt 6,0.

Daraus folgt, dass dieses Heilmittel die Proteindenaturierung nicht wie eine Säure sondern wie ein Präparat beeinflusst und die Polymerstruktur ändert.

Diese Eigenschaft gibt den therapeutischen Effekt dieses Heilmittels bei der Behandlung von z. B. malignen Tumoren vor.

### Beispiel 4

Das Blut der Sarkoma-Kranken (10 ml) wurde zentrifugiert. Ein Teil des gewonnenen Plasmas wurde getrocknet und von einem Polarisationsmikroskop aufgenommen. Der andere Teil desselben Plasmas wurde mit einer Wasserlösung Malachitgrün ergänzt. Die resultierende Mischung wurde ebenfalls im Polarisationsmikroskop aufgenommen.

Ergebnisse: In der ersten Mischung lag eine Lichtstreuung vor. In der zweiten Mischung wurde eine doppelte Lichtbrechung beobachtet.

Diese Angaben besagen, dass dieses Heilmittel das beim Krebs geänderte Folding der Proteine retabliert.

### Beispiel 5

Das Filtrat aus einem Melanom-16 (10 ml) wurde in eine Quartzküvette gesetzt und danach von einem Polarisationsmikroskop aufgenommen.

Danach wurde dieses Filtrat mit einer Lösung von Malachitgrün ergänzt und wiederholt in Polarisationslicht aufgenommen.

Ergebnisse: Auf den Aufnahmen ohne Präparat ist die Lichtpolarisation gering. Auf den Aufnahmen nach der Injektion des Präparats wird eine ausgeprägte Polarisation des durch die Mischung passierten Lichts festgestellt.

Diese Angaben zeugen davon, dass dieses Heilmittel Strukturänderungen in den Krebszellenproteinen herbeiführt.

### Behandlungsbeispiele

### Beispiel 6

Kranker V. 49 J. Diagnose: Adenokarzinom der Lungen. Metastasen im Gehirn. Kachexie. CT zeigt einige tumorartige Bildungen im Mittelfell und in der rechten Lunge. Die tumorartigen Bildungen haben eine durchschnittliche Abmessung von 2,7 x 2,9 x 3,0 cm. Schwerer Zustand, Atemnot, Zyanose, Adynamie, Schmerzempfindungen, die beim Husten stärker werden. Die verordnete Behandlungskur umfasste die Anwendung des Arzneimittels mit antiblastomatöser Wirkung. Die erste Behandlung mit dem Präparat umfasste die Einnahme (oral) von je 15 Tropfen der 1%igen Lösung / Glas Wasser dreimal am Tag; am Mittag zusätzlich ein Mikroklistier ä 10 Tropfen der 1%igen Lösung / 30 ml. Wasser; und für die Nacht 1 %ige Zäpfchen mit dem Präparat. Im Laufe der Woche: Im Wesentlichen ohne Änderungen; jedoch wurde vom Patienten bemerkt, dass innerhalb von einigen Stunden nach der Behandlung das Schmerzensyndrom beachtlich nachlässt. In der dritten Woche nach dem Behandlungsanfang wurde der Zustand des Kranken besser. Drei Monate später (ab Behandlungsanfang) werden auf der CT keine Metastasen im Gehirn mehr erkannt. Der Lungentumor verkleinerte sich bis auf 1,3 x 1,2 x 1,2 cm. Nach 6 Monaten der Behandlung verringerte sich der Tumor bis auf 0,5 x 0,7 x 2,0 cm. Es werden keine Metastasen im Gehirn mehr beobachtet. Die Atemnot hat abgenommen und die Schmerzen sind praktisch verschwunden. Das Blutbild wurde im Laufe der Behandlung normalisiert.

### Beispiel 7

Kranker T. 33 J. Vor drei Jahren wurde die rechte Niere entfernt und eine vorbeugende Chemotherapie im Zusammenhang mit klarzelligem Krebs vorgenommen. Bei der Untersuchung mittels CT wurde in der Projektion der rechten Niere tumorähnliche Bildung mit Abmessungen von 2,5 x 4,4 x 3,2 cm sowie drei runde tumorähnliche Bildungen mit einem Durchmesser von 3,0 bis 3,3 cm in der linken Lunge beobachtet. Schwerer Zustand, Atemnot, Zyanose, Adynamie.

Die verordnete Behandlungskur umfasste die Anwendung von einem Arzneimittel mit antiblastomatöser Wirkung. Das Präparat Malachitgrün wurde peroral in Form von einem Getränk ä 16 Tropfen der 1%igen Lösung dreimal am Tag und sowie in Form von Mikroklistieren eingeführt. Nach zwei Wochen der Behandlung nahmen die Atemnot sowie das Schmerzsyndrom ab. Das Blutbild wurde im Laufe der Behandlung innerhalb von 3 Wochen normalisiert. Danach wurde die wiederholte Behandlungskur mit dem Präparat vorgenommen. Das Präparat wurde per rectum in Form von 1%igen Zäpfchen und inhalatorisch im Laufe von 6 Monaten eingeführt. Während der Therapie sind der Tumor in der rechten Niere und die Metastasen in den Lungen verschwunden. Das Gewicht ist wiederhergestellt worden.

### Beispiel 8

Kranker B. 62 J. Diagnose: Harnblasenkrebs. Metastasen in die Beckenknochen. Vor drei Jahren wurde eine Blasenresektion wegen Krebs vorgenommen. Beschwerden über Schmerzen, Hämaturie und häufiges Harnlassen. Die CT zeigt eine tumorähnliche Bildung von 1 x 2 x 1 cm in der Mündung der Projektion des rechten Harnleiters. in der rechten Darmbeinschaufel waren zwei tumorähnliche Bildungen mit unebenen Umrissen, mit einem Durchmesser von 1 bis 2 cm. Mittelschwerer Zustand, blasse Hautdecken. Die Behandlung mit dem Präparat wurde nach folgendem Ablauf angefangen: 20 Tropfen der 1%igen Lösung Malachitgrün per halbes Glas peroral und in Form von Mikroklistieren. Die Behandlungskur dauerte 2 Monate. Während der Behandlung verringerte sich der Tumor bis auf 0,2 x 0,2 x 0,2 cm. Die Metastasen in den Beckenknochen verringerten sich um 1,5 cm. Es wurden Biophosphonate zur Substitutionstherapie verordnet. In der zweiten Woche ab Anfang der Behandlung ließen die Blutungsbereitschaft und das Schmerzsyndrom nach. Das Blutbild wurde im Laufe der Behandlung innerhalb von 4 Wochen normalisiert. Die volle Behandlungskur mit dem Präparat dauerte 6 Monate. Während der Therapie verschwanden der Tumor in der Harnblase sowie die Metastase im Becken. Das Gewicht ist wiederhergestellt worden.

### Beispiel 9

Kranker B. 23 J. Diagnose: Glioblastom des Gehirns rechts.

Rezidiv. Vor zwei Jahren wurde die Tumorexstirpation wegen Glioblastoms des Gehirns vorgenommen. Beschwerden über Kopfschmerzen, Übelkeit, Erbrechen. Die Kernspintomografie lässt in der Hirnsubstanz in der Projektion der Operationsnarbe einen Tumor mit unebenen Kanten von 2,2 x 2,4 cm erkennen. Mittelschwerer Zustand, blasse Hautdecken. Dem Patienten wurde die Behandlung mit dem Präparat verordnet: Das Malachitgrün 500 mg pro 500 ml. der physiologischen Lösung steril wurde intravenös als Tropfeninfusion gegeben. Die Prozedur dauerte 60 Minuten lang. Die nachfolgenden Prozeduren wurden in der gleichen Reihenfolge zweimal wöchentlich durchgeführt. Die Behandlungskur dauerte 2 Monate. Während der Behandlung verringerte sich der Tumor bis auf 0,1 x 0,2 cm. Nach zwei Infusionen des Präparats ließen die Übelkeit und das Schmerzsyndrom nach. Alle zwei Wochen im Laufe von 6 Monaten wurde eine einmalige Präparat-Injektion durchgeführt. Nach 8 Behandlungsmonaten lässt sich auf der CT kein Tumor mehr erkennen.

### Beispiel 10

Kranker L. 64 J. Diagnose: Hepatom. Primärer Leberkrebs. Vor 2 Jahren wurde die Resektion von einem Teil des mit Tumor betroffenen rechten Leberlappens wegen Hepatoms vorgenommen. Beschwerden über Schmerzen im rechten Hypochondrium, Atemnot, Aszites, wiederkehrende Stuhlverhaltung. Mittelschwerer Zustand, schwacher Appetit, Gelbsüchtigkeit der Hautdecken. Auf dem US-Bild lässt sich ein Tumor im linken Leberlappen mit unebenen Kanten von 3 x 4 cm erkennen. Es wurde die Bougierung der Nabelvene vorgenommen. Das sterile Präparat Malachitgrün wurde im Laufe von zwei Wochen zweimal am Tag mit einem Katheter eingeführt und zwar: à 100 mg von Malachitgrün aufgelöst in 30 ml einer physiologischen Lösung. Die nachfolgenden Prozeduren wurden in derselben Reihenfolge 3-mal wöchentlich durchgeführt. Dabei wurde das Präparat nur intravenös gegeben. Die Behandlungskur dauerte 2 Monate. Während der Behandlung verringerte sich der Tumor im betroffenen Gebiet bis zu Abmessungen von 0,3 x 0,2 cm. 4 Monate später blieb an der Stelle des Tumors in der Leber nur eine höchstwahrscheinlich Bindegewebsnarbe von 0,1 x 0,1 cm übrig. Im Laufe der Behandlung unter Einsatz des Präparats wurde eine positive Entwicklung im Blutbild festgestellt.

### Ergebnisse der Gesamtblutuntersuchung

| **Hauptkennziffern** | **10.12.03** | **29.12.03** | **04.02.04** | **07.04.04** |
|---|---|---|---|---|
| Erythrozyte | 3,22 | 3,60 | 4,37 | 4,67 |
| Hämoglobin | 74 | 80 | 117 | 123 |
| Thrombozyte | 315 | 362 | 286 | 310 |
| Leukozyte | 7,2 | 7,8 | 5,8 | 6,7 |

| **Hauptkennziffern** | | **10.12.03** | **29.12.03** | **04.02.04** | **07.04.04** |
|---|---|---|---|---|---|
| Basophile | 0 - 1 % | 1 | - | - | 1 |
| Eosinophile Leukozyte | 0 - 5 | 1 | 2 | 2 | 1 |
| Nuclear bacilli | 1 - 6% | 1 | 1 | - | - |
| Kernsegmente | 45 - 70 | 2 | 4 | 6 | 5 |
| Shift indexes | | 39 | 43 | 56 | 67 |
| Lymphozyte | 18 - 40% | 9 | 13 | 28 | 17 |
| Monozyte | 2 - 9% | 15 | 16 | 37 | 39 |
| ESR 1 - 15 mm/h | | 3,5 | 4,5 | 5,0 | 8,5 |

### Ergebnisse der biochemischen Blutuntersuchung

| **Hauptkennziffern** | **Norm** | **10.12.03** | **20.12.03** | **04.02.04** | **07.04.04** |
|---|---|---|---|---|---|
| Gesamteiweiß | 65,0 - 85,0 gl [*g*/*l*?] | 44,43 | 45,90 | 68 | 65,64 |
| | | 98,0 | 83 | 79 | 67 |
| Kreatinin | 44-110 | 7,2 | 6,4 | 4,1 | 2,6 |
| Harnstoff | 1,66 - 8,3 | 6,21 | 5,62 | 5,75 | 7,03 |
| Cholesterin | 2,90 - 6,54 | 11,0 | 24,2 | 8,4 | 8,0 |
| Bilirubin, gesamt | 8,5 - 20,5 | 3,0 | 22,3 | 3,3 | 2,0 |
| | Bis 25 % | 8,0 | 11,9 | 5,1 | 6,0 |
| Bilirubin, direktes | Bis 25 % | 82 | 96 | 84 | 90 |
| | Bis 100 EI | 107,2 | 102 | 89 | 78,0 |
| Bilirubin, indirektes | 95 - 100,0 | 114,0 | 140 | 102,8 | 110,0 |
| | 135,0 - 147,0 | 114,20 | 115,3 | 114,31 | 134,2 |
| Amylase | | 3,8 | 4,9 | 4,8 | 4,9 |

| **Hauptkonnziffern** | **Norm** | **10.12.03** | **20.12.03** | **04.02.04** | **07.04.04** |
|---|---|---|---|---|---|
| Chlor | 3,5-6,0 | 2,72 | 2,32 | 2,28 | 2,15 |
| Natrium | 0,98 - 1,30 | 19,4 | 13,4 | 18,0 | 17,7 |
| Kalium | 2,1-2,55 | 314,3 | 395,8 | 138,0 | 64,4 |
| Kalzium | zh [?] 8,8 - 27,0 | 190,4 | 224,7 | 94,4 | 43,7 |
| | 5 - 40 EI | | | | |
| | | 564,4 | 431,7 | 113,8 | 30,4 |
| Kalzium, gesamt | 5-40 EI | 765,8 | 526 | 254,2 | 48 |
| | zh bis 104 | 5,71 | 5,48 | 4,55 | 6,7 |
| Serumeisen | EI | 876 | 456 | 432 | 342 |
| AST | 225-450 | | | | |
| GPT | EI | | | | |
| Alkalische Phospha- | | | | | |
| tase | | | | | |
| LDH | | | | | |

Momentan liegen keine Beschwerden mehr vor.

### Beispiel 11

Kranker T. 58 J. Diagnose: Non-Hodgkin-Lymphom. Kachexie. Vor 1,5 Jahren wurde eine Vollkur von Polychemotherapie durchgeführt. Vor einem halben Jahr entstanden geschwulstähnliche Bildungen auf dem Hals und unter dem Kiefer. Auf dem Röntgenbild lässt sich eine Mittelfellerweiterung erkennen. Zur Zeit der Einweisung: Beschwerden über Atemnot, Schwäche, vermehrte Schweißabsonderung. Mittelschwerer Zustand, Unterernährung, blasse Hautdecken. Im Submandibulargebiet links befand sich eine tumorähnliche Bildung von 2 x 3 cm. Auf dem Hals gab es beidseitig geschwulstähnliche Bildungen mit Abmessungen von jeweils 2 x 3, 3 x 4 cm. Die Behandlung mit dem Präparat Malachitgrün erfolgte gemäß dem folgendem Ablauf: 20 Tropfen der 1%igen Lösung von Malachitgrün aufgelöst in 150 ml Wasser wurden peroral dreimal am Tag eingenommen. Die Behandlungskur dauerte 4 Monate. Nach 12 Tagen ab Behandlungsanfang sind die submandibularen Halslymphknoten beim Patienten verschwunden. Innerhalb von einem Monat verkleinerten sich die Geschwülste im Halsgebiet bis auf 0,3 x 0,4 cm. Nach 3 Monaten ließ sich keiner der Halslymphknoten mehr wieder erkennen. Das Blut- und Harnbild sind in Ordnung. Das Gewicht ist wiederhergestellt. Es liegen keine Beschwerden mehr vor.

### Beispiel 12

Kranker B. 45 J. Diagnose: Dickdarmadenokarzinom. Metastasen in die Leber. Aszites. Vor 3,5 Jahren wurde eine Dickdarmresektion mit End-zu-End-Anastomose vorgenommen. Der Patient hat eine volle Polychemotherapie durchgemacht. 2 Jahre nach der Behandlung entstanden Schmerzen in der unteren Bauchpartie, Tenesmus. Zur Zeit der Einweisung Beschwerden über akute Schwäche, ständige Bauchschmerzen, Husten, Atemnot, Aszites, Stuhlverhaltung. Schwerer Zustand, Unterernährung, blasse Hautdecken mit zyanotischer Tönung, Asziteszeichen im Bauch. Im Blutbild wurde der Rückgang an Erythrozytenmenge, Hämoglobin, Leukozyten, Albumin, die Erhöhung von GPT und AST durchgeführt. Bei der Computertomographie lässt sich im rechten Leberlappen eine Bildung mit einer unregelmäßigen Form von 2 x 4 cm mit unebenen Kanten erkennen. Im Anastomosegebiet wird eine voluminöse Bildung von 4 x 5,4 cm sowie eine ungebundene Flüssigkeit in der Bauchhöhle beobachtet. Das Präparat Malachitgrün wurde dem Patienten in Form von intravenösen Infusionen, Mikroklistieren und Getränken verordnet. Die Infusionslösung stellt 200 mg des sterilen Malachitgrüns aufgelöst in 500 ml der physiologischen Lösung dar. Die Infusionen wurden wöchentlich innerhalb von 3 Monaten durchgeführt. Die Mikroklistiere sind Mischungen von 20 Tropfen der 1%igen Lösung von Malachitgrün in 30 ml gekochtem Wasser. Die Mikroklistiere wurden dreimal am Tag im Laufe von 2 Monaten gemacht. Um den Therapieeffekt zu erhöhen, wurde das Präparat in Form der 1%igen Lösung Malachitgrün in 200 ml gekochtes Wasser 20 Minuten vor dem Essen dreimal am Tag eingenommen. Nach 10 Tagen ab Behandlungsanfang sind die Schmerzen beim Kranken völlig verschwunden. Der Appetit kam wieder. In der dritten Behandlungswoche wurde die Atemnot geringer. Während der Behandlung verkleinerte sich der Tumor im Anastomosegebiet bis auf 0,2 x 0,3 cm. 3 Monate später wurde eine Pause von einem Monat gemacht und danach wurde die Therapie für weitere 2 Monate fortgesetzt. Während der Behandlung werden keine Metastasen in der Leber sowie kein Tumor im Anastomosegebiet wiedererkannt. Es wird eine allmähliche stufenweise Verbesserung der Blutkennwerte bemerkt, was direkt mit der Behandlung mit dem Präparat zusammenhängt.

### Ergebnisse der Blutuntersuchung

| **Hauptkennziffern** | **09.10.04** | **24.11.04** | **09.12.04** | **01.09.05** |
|---|---|---|---|---|
| Erythrozyte | 2,80 | 3,84 | 4,44 | 4,62 |
| Hämoglobin | 74 | 110 | 115 | 128 |
| Thrombozyte | 320 | 310 | 326 | 440 |
| Leukozyte | 9,2 | 12,0 | 5,2 | 5,3 |
| Basophile 0 - 1 % | 1 | - | - | 1 |
| Eosinophile Leukozyte 0 - 5 | 1 | 2 | 2 | 1 |
| Nuclear bacilli 1 - 6 % | 0 | 1 | - | 2 |
| Kernsegmente 45 - 70 | 2 | 6 | 6 | 5 |
| Lymphozyte 18 - 40 % | | | | |
| Monozyte 2 - 9% | 10 | 24 | 32 | 39 |
| ESR 1 - 15 mm/h | 3,2 | 3,7 | 5,1 | 5,9 |

| **Ergebnisse der biochemischen Blutuntersuchung** | | | | | |
|---|---|---|---|---|---|
| **Main Indexes** | **Norm** | **09.10.04** | **24.11.04** | **09.12.04** | **01.09.05** |
| Gesamteiweiß | 65,0 - 85,0 gl | 50,30 | 60,19 | 69 | 83,52 |
| | | 118,0 | 89 | 74 | 69 |
| Kreatinin | 44-110 | 8,2 | 7,4 | 3,1 | 2,9 |
| Harnstoff | 1,66 - 8,3 | 6,21 | 5,62 | 5,75 | 7,03 |
| Cholesterin | 2,90 - 6,54 | 11,0 | 24,2 | 8,4 | 8,0 |
| Bilirubin, gesamt | 8,5 - 20,5 | 3,0 | 22,3 | 3,3 | 2,0 |
| | Bis 25 % | 8,0 | 11,9 | 5,1 | 6,0 |
| Bilirubin, direktes | Bis 25 % | 82 | 96 | 84 | 90 |
| | Bis 100 EI | 107,2 | 102 | 89 | 78,0 |
| Bilirubin, indirek- | 95 - 100,0 | 114,0 | 140 | 102,8 | 110,0 |
| tes | 135,0 - 147,0 | 114,20 | 115,3 | 114,31 | 134,2 |
| Amylase | | 3,8 | 4,9 | 4,8 | 4,9 |
| Chlor | 3,5 - 6,0 | 2,72 | 2,32 | 2,28 | 2,15 |
| Natrium | 0,98 - 1,30 | 19,4 | 13,4 | 18,0 | 17,7 |
| Kalium | 2,1 - 2,55 | 314,3 | 395,8 | 138,0 | 64,4 |
| Kalzium | zh 8,8 - 27,0 | 190,4 | 104,7 | 54,8 | 42,9 |
| | 5 - 40 EI | 165,7 | 145,8 | 45,7 | 29,5 |
| Kalzium, gesamt | 5 - 40 EI | | | | |
| | zh bis 104 | 465,8 | 326 | 159,2 | 79 |
| Serumeisen | EI | 523 | 432 | 342 | 228 |
| AST | 225-450 | | | | |
| GPT | EI | | | | |
| Alkalische Phosphatase | | | | | |
| LDH | | | | | |

Das Gewicht ist wiederhergestellt worden. Innerhalb von 2 Jahren liegen keine Beschwerden mehr vor.

### Beispiel 13

Kranker K. 65 J. Bei der Einweisung: Beschwerden über schneidende Schmerzen beim Harnlassen sowie über Harnsperre und -rötung. Bei der Untersuchung: US-Untersuchung vom 04.02.2001 - Vorsteherdrüse = 93 cm³, Tumor mit unscharfen Konturen von 32 x 42 mm². PSA am 02.02.2001 - 49,9 mg/ml. Punktionsbiopsie - Adenokarzinom der Vorsteherdrüse.

Diagnose: Adenokarzinom der Vorsteherdrüse. Der Patient hat die Operation und die Chemotherapie abgesagt.

Es wurde die Behandlung mit dem Arzneimittel Malachitgrün vorgenommen.

Ab 09.03.2001 im Laufe von 20 Tagen nahm der Patient das Präparat Malachitgrün à 300 mg x 2 mal am Tag beim Essen und 30 Minuten nach dem Essen - Malachitgrün ä 200 mg ein. Für die Nacht wurde ein Zäpfchen mit 2%igem Präparat eingeführt. Innerhalb dieser Zeit nahmen die Schmerzen beim Harnlassen ab. Die Harnfarbe normalisierte sich. Der Umfang der Vorsteherdrüse verringerte sich bei der US-Untersuchung um 40 %. Der Tumor bekam scharfe Umrisse mit Abmessungen von 13 x 14 mm² Danach wurde die Therapie mittels der Einnahme des Präparats Malachitgrün innerhalb von zwei Wochen à 300 mg x 2 mal am Tag beim Essen durchgeführt. Es wurden auch die Heilklistiere täglich vor dem Schlaf ä 2,0 g von Malachitgrün in 50 ml gekochtem Wasser verordnet.

Bei der nachfolgenden Untersuchung waren, so der Patient, die Kennzeichen der Krankheit (schneidende Schmerzen beim Harnlassen sowie Harnsperre usw.) völlig verschwunden. Auf der folgenden US-Aufnahme hat die Vorsteherdrüse den Umfang von 45 cm³, der Tumor verringerte sich bis auf 9 x 5 mm². PSA am 09.05.2001 - 1,1 mg/ml. Im Laufe von 3 Jahren wurde kein Rezidiv festgestellt.

### Beispiel 14

Kranke T., 52 J. 2001 wurde die Gebärmutter- und Anhängerexstirpation im Zusammenhang mit der Sichtbarmachung des Karzinoms des linken Eierstocks vorgenommen. Während 2 Jahren nach der Operation wurden Chemotherapien durchgeführt.

Ende Juli 2002 entstanden Schmerzen im Mastdarm und Tenesmus. Auf dem CT vom 12.09.2001 ließ sich ein Tumor mit Abmessungen von 13,0 x 12,5 cm zwischen dem Mastdarm und der Harnblase erkennen. Der Tumor wuchs durch ihre Wände hindurch.

Diagnose: Rezidivkarzinom des linken Eierstocks. Angesichts der Wirkungslosigkeit der weiteren Chemotherapie wurde die Behandlung mit dem Präparat in Form von Klistieren durchgeführt. Am Anfang der Behandlung, innerhalb von 21 Tage wurde das Malachitgrün à 300 mg pro Tag eingenommen.

Am 3. Tag nach dem Anfang der Behandlung nahmen die Schmerzen ab. Die Harn-und Defäkationsdränge wurden seltener. US-Untersuchung 2 Wochen später: die Tumorabmessungen betrugen 4,2 x 3,0 cm², noch 4 Wochen später - 1,4 x 1,2 cm². Es ließ sich eine scharfe Grenzlinie zwischen dem Tumor und den Wänden von Mastdarm und Harnblase erkennen.

Nach 3 Monaten wurde auf der US-Kontrollaufnahme eine runde Bildung mit scharfen Grenzen und Abmessungen von 0,4 x 0,5 cm² erkannt.

Bei einer zusätzlichen Untersuchung war der Zustand des Patienten zufriedenstellend. Auf der US-Aufnahme vom 21.03.2005 wurde eine Bildung von 0,4 x 0,3 cm² mit scharfen Umrissen beobachtet. Momentan ist die Patientin gesund.

### Beispiel 15

Kranker T. 45 J. Diagnose: Klarzelliger Krebs der rechten Niere. Metastasen in die Lungen.

Der Patient machte einige Chemotherapien und lmmunotherapien durch. Der Tumor und die Metastasen wuchsen weiter. Es wurde die Behandlung mit Malachitgrün à 50 mg, aufgelöst in 150 ml Wasser dreimal am Tag 20 Minuten vor dem Essen vorgeschlagen. Es wurden auch 5%ige Zäpfchen per rectum für die Nacht eingesetzt. Die Behandlung nach einem solchem Plan erfolgte im Laufe von 3 Monaten.

4 Monate später wurde kein ursprünglicher Tumor auf dem CT festgestellt. Die kleinen Metastasen in der rechten Lunge wiesen Kalzinierungskennzeichen auf.

### Beispiel 16

Kranke Sch. 75 J. Diagnose: Hautkrebs. Im rechten Unterschenkelbereich befand sich die Herdaffektion mit Abmessungen von 12 x 15 x 12 cm. Gistologische Diagnose: Hautkrebs. Es wurde die Behandlung mit 2%iger Salbe auf Malachitgrün-Besis vorgenommen. Innerhalb von 2 Monaten verringerte sich der Tumor um 70 %. 3 Monate später wurde an der ehemaligen Tumorstelle eine Überpigmentierung beobachtet. Die Patientin hat keine Beschwerden. Innerhalb von 3 Jahren erfolgten keine Rezidive.

Damit ist ein Heilmittel gefunden, welches ein Breitspektrum der Heilwirkung auf verschiedene Geschwulstkrankheiten aufweist. Dabei wird ein weit und breit zugängliches und kostengünstiges Mittel zur Behandlung von Geschwulstkrankheiten verwendet. Dieses Heilmittel ist nichttoxisch, hat keine Nebenwirkung bei seiner Anwendung in pharmazeutisch zulässigen Dosierungen. Dieses Heilmittel stellt kürzere Behandlungsdauer sowie die Erhöhung der Behandlungseffektivität sicher.

## Patentansprüche

1. Anwendung von Malachitgrün in Form von ANHYDROOXALAT TETRAMETHYLDIAMINTRIPHENYLKARBINOL (ANHYDROOXALATE TETRAMETHYLDIAMISHTRIPHENOLCARBINOL) [(C₂₃H₂₅N₂)-(C₂HO₄)]₂-C₂H₂O₄ mit folgender Bauformel: 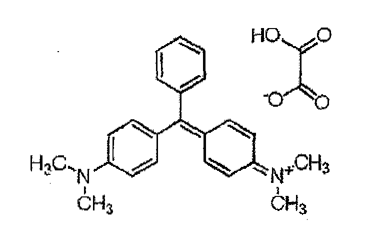 als Mittel zur Behandlung von malignen Neubildungen mit einer Dosierung des Arzneimittels von 1 mg bis 2 g pro Einnahme.

2. Anwendung nach Anspruch 1,
**dadurch gekennzeichnet,**
**dass** das Malachitgrün in Form einer Wasserlösung oder einer Lösung in einer physiologischen Kochsalzlösung oder im Alkohol eingesetzt wird.

3. Anwendung nach Anspruch 2,
**dadurch gekennzeichnet,**
**dass** die Wasserlösung von Malachitgrün peroral vor oder nach dem Essen eingenommen wird.

4. Anwendung nach einem der Ansprüche 1, 2,
**dadurch gekennzeichnet,**
**dass** die Lösung von Malachitgrün rektal eingeführt wird.

5. Anwendung nach einem der Ansprüche1, 2,
**dadurch gekennzeichnet,**
**dass** eine 1 %ige Lösung von Malachitgrün intravenös injektiert wird.

6. Anwendung nach Anspruch 1,
**dadurch gekennzeichnet,**
**dass** das Malachitgrün rektal in Form von Zäpfchen angewendet wird.

7. Anwendung nach Anspruch 1,
**dadurch gekennzeichnet,**
**dass** das Malachitgrün als ein Bestandteil einer 1 - 5 % Salbe angewendet wird.
